# EUROPEAN PATENT SPECIFICATION

(11) **EP 0 627 211 B1**
(45) Date of publication and mention of the grant of the patent: **25.08.1999**
(21) Application number: 94108466.7
(22) Date of filing: 01.06.1994
(51) Int. Cl.: A61F 13/15

(54) **Apparatus for making absorbent products containing a first material dispersed within a second material**
Vorrichtung zum Herstellen von absorbierenden Erzeugnissen die ein in einem zweiten Material dispergiertes Ausgangsmaterial enthalten
Appareil pour la production de produits absorbants contenant une matière dispersée d'une autre matière

(30) Priority: 02.06.1993 US 71632
(43) Date of publication of application: 07.12.1994
(73) Proprietor: McNEIL-PPC, INC., Milltown New Jersey 08850 (US)
(72) Inventor: Griffoul, Thomas, Jamesburg, NJ 08831 (US); Wislinski, Martin, Edison, NJ 08820 (US)
(74) Representative: Groening, Hans Wilhelm, Dipl.-Ing.

(56) References cited:
- EP-A- 0 313 800
- EP-A- 0 453 892
- WO-A-88/04165
- US-A- 4 674 966
- US-A- 4 927 582

## Description

### Field of the Invention

The current invention is directed to an apparatus for making absorbent products, such as sanitary napkins and the like, containing a first material dispersed throughout at least a portion of a second material. More specifically, the current invention is directed to the manufacture of an absorbent product having two zones -- one containing pure fluff and the other containing a fluff dispersed particulate, such as superabsorbent particles.

European patent application 0 313 800 discloses features contained in the preamble of claim 1 and is related to a hygienic article made of cellulose as a disposable article and a method for making it. Articles of this kind, like diapers, comprise a superabsorbent body to which superabsorbent substances have been added. This document is based on the object to provide an optimal dispersion of the absorbent components of the diaper corresponding substantially to the profile of disperse of the fluid within the diaper and having an optimal fluid receiving capacity. In addition to an economic use of the superabsorbent substances it is intended to locate most of the absorbent substances in that area of the diaper which receives most of the fluid. An apparatus for manufacturing these diapers has a shroud as a guidance for a first airstream. At the end of this shroud there is mounted a drivable screen drum being partially enclosed by the shroud. The circumferential edge portion projects from both of its side faces. The drum is provided with molds which are separated by webs and receives mixed substances supplied by the first and second airstream. The second airstream is vertically directed into the first airstream carrying the superabsobent substances. The mixed airstreams are drawn by a vacuum source through the circumference of the drum so that the mixture of substances carried therewith are backheld at the periphery of the drum.

US-A-4 674 966 discloses an apparatus for forming fibrous pads in the shape in which they will be used, without the need for later cutting and, if desired, with varied thickness over its surface and by the use of an apparatus which vacuum-forms fibrous pads by use of interchangeable inserts having a screen, supported by a foraminous floor which moves along with the screen to minimize wear to the screen. This known apparatus comprises a hammermill to defiberize web of compressed fibrous material so that the fibers entrained in an airstream caused by a vacuum source can be drawn down onto depressions on the upper surfaces of said inserts having the shape and contour of the pad desired. Said insert pass under scarfing rolls where the excess fibers are taken off and recycled via a stack thereafter said inserts and the pads therein pass under another take-off roll, where the pads are taken from the inserts and dropped on a conveyor for wrapping and packaging. A shroud surrounds the entire upper portion of the drum. The purpose of the shroud is to provide an area through which the fluff can be dispersed before it is drawn into the inserts and to close the vacuum parts around the inserts. The scarfing rolls remove the extra fluff from each pad being piled above the upper surfaces of the inserts so that the top of the pad is even with the top surface of the insert. The scarfing rolls accomplish this function by spinning at high speed in the same direction as the drum such that the surface of the roll wraps against the upper surface of inserts and removes the extra fluff therefrom, which is recirculated to the hammermill via said stack. A take-off roll is positioned in an area where there is no vacuum behind the inserts of the drum so that the pads are taken out from the inserts by said take-off wheel because of a vacuum behind it. In a portion where the pads are no longer held by the take-off wheel the pads drop onto a conveyer to be conveyed to a downstream processing machinery for wrapping and packaging.

WO-A 88 04165 discloses a method and apparatus for forming a non-woven pad consisting of fibrous material in which highly moisture-absorbing particles are intermixed with the fibrous material throughout a predetermined portion of the thickness of the non-woven pad. The non-woven pad is formed atop a conveyor moving through a chamber which has a duct connected to a source of vacuum operable to draw fibrous material injected into the chamber onto the conveyor. A spray gun is positioned within the chamber relative to the fibrous material atop the conveyor and is operated to discharge moisture-absorbing material at a predetermined velocity , such that the moisture-absorbent material is intermixed with the fibrous material throughout preferably a center layer of the thickness of the non-woven pad while forming boundary layers on either side of the center layer which are substantially free of moisture-absorbent material. The spray gun preferably operates intermittently to form spaced, sharply defined areas along the length and width of the non-woven pad wherein each area has moisture-absorbent material interspersed throughout a portion of the thickness thereof. The objectives to be achieved by this structure are to minimize waste of the moisture-absorbent material to maximize the moisture-retaining capacity of the non-woven pad while limiting damage to die cutters and other apparatus in forming the finished hygienic article and to reduce contamination of the environment with oversprayed moisture-absorbent material.

Consequently, it would be desirable to provide an apparatus for making absorbent products comprised of first and second materials in which the second material (i.e., superabsorbent particles) was well distributed throughout the first material (i.e., absorbent fluff). It would also be desirable to provide an apparatus for making absorbent products comprised of first and second layers in which the first layer contained pure first material and the second layer contained a mixture of the first and second materials.

### Summary of the Invention

The present invention accomplishes this and other objects in an apparatus as disclosed in claim 1.

Claims 2 to 9 contain additional improvements of the invention as disclosed in claim 1.

### Brief Description of the Drawings

Figure 1 is an elevation, partially schematic, of the apparatus for making absorbent products according to the current invention.
Figure 2 is an isometric view of the forming wheel of the apparatus shown in Figure 1.
Figure 3 is a detailed view of the vacuum forming chamber portion of the apparatus shown in Figure 1.
Figures 4-6 are cross-sections taken through lines IV-IV, V-V, and VI-VI, respectively, shown in Figure 3, showing various stages of the product formation as the mold travels through the vacuum forming chamber.
Figure 7 is a cross-section through the completed absorbent core.

### Description of the Preferred Embodiment

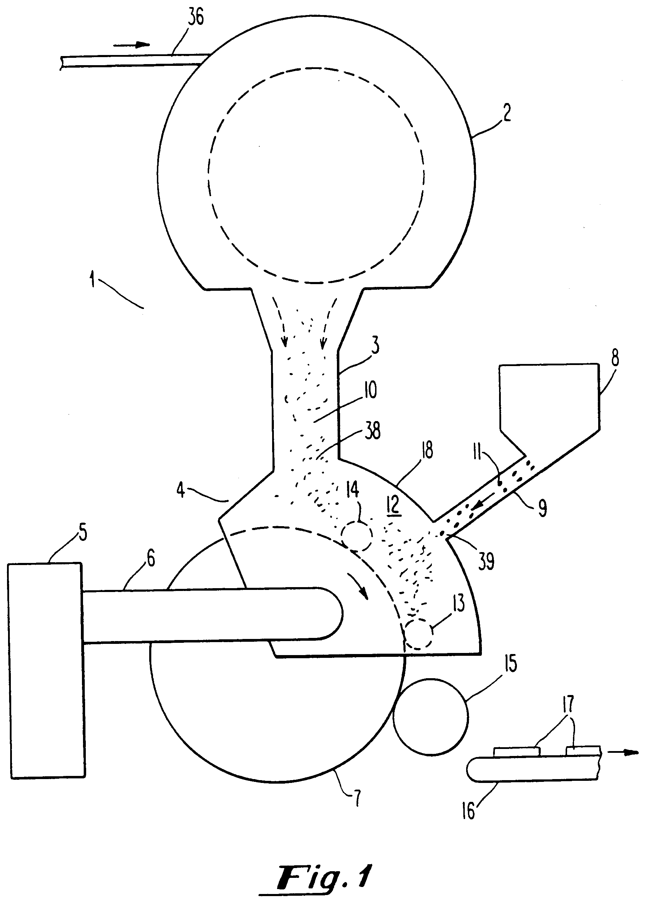

An apparatus 1 for making absorbent products according to the current invention is shown in Figure 1. The apparatus comprises a device to de-fiberize pulp, such as a hammermill 2, and a vacuum forming unit 4 that has been modified in accordance with the teachings of the current invention. Hammermills and vacuum forming units of the type shown in Figure 1 are described in detail in U.S. patent No. 4,674,966 (Johnson et al.) (assigned on its face to Winkler & Dunnebrier), U.S. patent No. 4,592,708 (Feist et al.) (assigned on its face to Procter & Gamble), and EPO published application No. 0,151,033 (Peterson et al.) (assigned on its face to Procter & Gamble).

As shown in Figure 1, a web of compressed fibers of an absorbent material 36, such as wood pulp, is fed into the hammermill 2. As is conventional, in the hammermill 2, the compressed fibrous material 36 is broken down into individual fibers, collectively referred to as "fluff" 10. The fluff 10 is directed via duct 3 into the vacuum forming unit 4 drawn by the suction created within the vacuum forming unit 4 by virtue of its holding a vacuum.

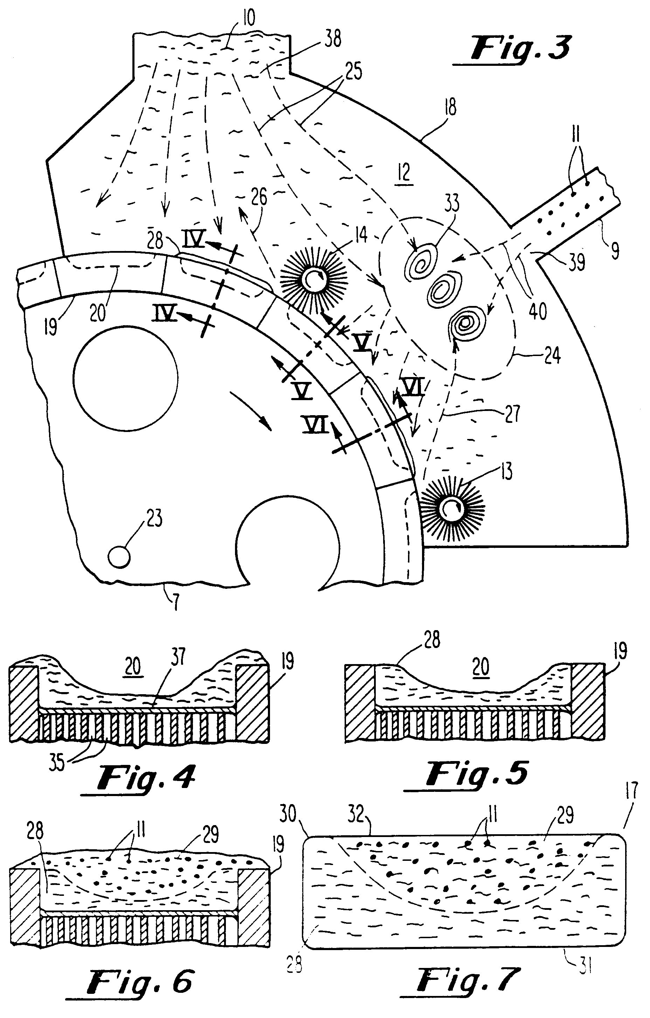

The vacuum forming unit 4 is comprised of a vacuum forming chamber 12 and a forming wheel 7. The vacuum forming chamber 12 is formed within a housing 18 that is sealed to prevent loss of vacuum, as in conventional. The vacuum forming chamber housing 18 has an inlet 38 that is in flow communication with the duct 3 from the hammermill 2 so as to receive the fluff 10 and direct it in streams 25, shown in Figure 3, into the vacuum forming chamber 12. A suitable vacuum forming chamber containing the features discussed above is disclosed in the aforementioned U.S. patent No. 4,592,708 (Feist et al.).

According to the current invention, the vacuum forming chamber housing 18 also has an inlet 39 that is connected via a duct 9 to a hopper 8 that contains a second material to be interspersed into the fluff 10. In the preferred embodiment, the second material comprises superabsorbent particles 11. Suitable superabsorbent particles are described in U.S. patent No. 4,540,454 (Pieniak et al.) (assigned on its face to Personal Products Company). However, the current invention could also be practiced using heat activated fibers or baking soda as the second material. Suitable heat activated fibers may be obtained from Hercules Corporation as PULPEX™.

The superabsorbent particles 11 are directed by the inlet 39 into the vacuum forming chamber 12 in streams 40, shown in Figure 3. In the preferred embodiment, gravity and suction from the vacuum forming chamber 12 are relied upon to drive the superabsorbent particles 11 into the vacuum forming chamber. However, the hopper 8 could also be pressurized if desired, to impart added velocity to the superabsorbent particle streams 40.

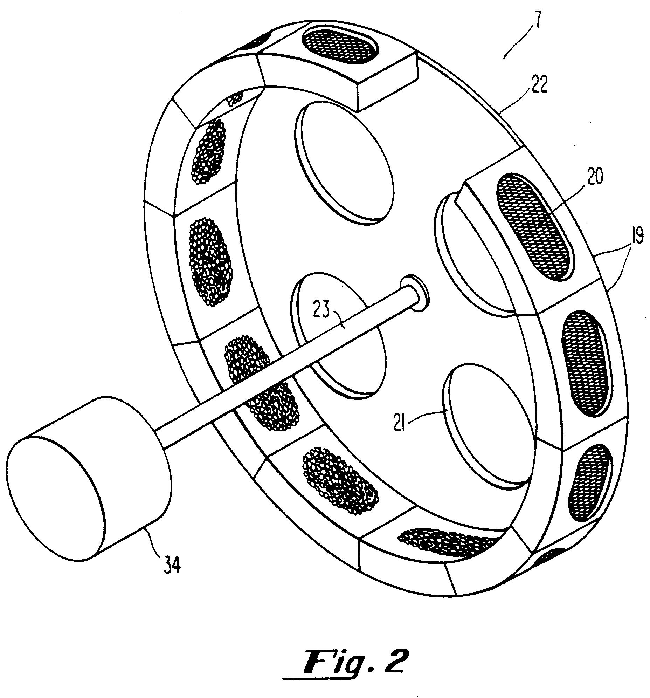

The forming wheel 7 is mounted for rotation within the housing 18 of the vacuum forming chamber 12 and places the vacuum forming chamber in flow communication with a duct 6 through which air is drawn by the vacuum source 5. As shown in Figure 2, a number of molds 19 are arranged circumferentially around the periphery of the forming wheel 7. In addition, the forming wheel 7 has a backing plate 22 in which passages 21 are formed that place the duct 6 from the vacuum source 5 in flow communication with the molds 19. A shaft 23 is mounted on the backing plate 22 and is coupled to a motor 34, via a speed reducing device (not shown), that drives the rotation of the forming wheel 7.

As shown in Figure 3, two scarfing brushes 13 and 14 are disposed within the vacuum forming chamber 12 and are located so as to scarf from the molds any fluff 10 that projects above the outward facing surface of the mold 19. As is conventional, the scarfing brushes 13 and 14 rotate in the same direction as the forming wheel 7.

As shown in Figures 2 and 4, each of the molds 19 has a cavity 20 that has the shape of the absorbent product to be made by the apparatus, in the preferred embodiment, the absorbent core of an absorbent product such as sanitary napkin, shown in Figure 7. A screen 37 is disposed in the bottom of the mold cavity 20 and rests atop the outlets of a number of air passages 35 formed within the body of the mold 19. Thus, the vacuum source 5 draws air from the vacuum forming chamber 12 by causing it to flow through the cavity 20, screen 37 and passages 35 of each of the molds 19.

Returning to Figure 1, a take-off wheel 15 removes the absorbent products 17 from the molds 19, after they have exited from the vacuum forming chamber 12, and deposits them onto a conveyor 16 for further processing. In the case of a sanitary napkin, such further processing would include the application of a fluid permeable cover and a fluid impervious barrier to the body facing side 31 and garment facing side 32, respectively, shown in Figure 7, of the product.

With reference to Figure 3, the operation of the apparatus 1 will now be explained in detail. As each mold 19 enters the vacuum forming chamber 12 and begins its passage through it, as a result of the rotation of the forming wheel 7, air is drawn through the molds by the vacuum source 5. As they enter the vacuum forming chamber 12, the molds are first placed into proximity with the fluff inlet 38. As a result, fluff 10 from the incoming streams 25 is gradually deposited within the mold cavities 20 and onto the mold surfaces. As shown in Figure 4, this results in a mold cavity 20 that is partially filled with a bottom layer of pure fluff 10.

Further rotation of the forming wheel 7 brings the molds 19 into contact with the first scarfing brush 14, which scarfs excess fluff 10 that projects above the surface of the mold and directs it in a stream 26 into the incoming streams 25 of fluff. As a result, a mold 19 that is clean shaven but still only partially filled with a layer 28 of pure fluff 10, as shorn in Figure 5, exits from the scarfing brush 14.

For reasons that will become apparent, it is important that the forming wheel 7 rotate with sufficient speed so that the cavity 20 is only partially filled by the time the mold exits the scarfing brush 14 and begins being filled with a mixture of fluff and superabsorbent particles from a mixing zone 24, as discussed further below. In the preferred embodiment, the forming wheel is rotated with sufficient speed that the cavity 20 is only about half filled by the layer 28 of pure fluff 10 before the deposition of the fluff/superabsorbent particle mixture begins.

Continued rotation of the forming wheel 7 brings the molds into proximity with the superabsorbent particle inlet 39 and, more significantly, with a mixing zone 24 in which superabsorbent particles 11 and fluff 10 are mixed. According to an important aspect of the current invention, the fluff inlet 38 and the superabsorbent particle inlet 39 are spaced around the periphery of the vacuum forming chamber housing 18 so that a portion of the incoming streams 25 of fluff 10 and a portion of the incoming streams 40 of superabsorbent particles 11 collide in a discrete area, referred to as the mixing zone 24, of the vacuum forming chamber 12 just downstream of the scarfing brush 14.

Streams 27 of fluff/superabsorbent particle mixture from the mixing zone 24 are drawn to the partially filled mold cavity 20 so as to completely fill it with a layer 29 of the mixture, as shown in Figure 6. Next, the mold 19 is rotated under the second scarfing brush 13, which rotates in the same direction as scarfing brush 14 and scarfs any excess fluff/superabsorbent particle mixture that extends above the surface of the mold.

According to an important aspect of the current invention, the scarfing brush 13 is located so that it directs the scarfed fluff/superabsorbent particle mixture in a stream 27 back into the mixing zone 24. In the preferred embodiment, the scarfing brushes 13 and 14 are each about 101.6mm (four inches) in diameter and are spaced about 20° apart (i.e., about 203.2mm (eight inches)). By directing the scarfed fluff/superabsorbent particle mixture into the mixing zone 24 turbulence, indicated by reference numeral 33 in Figure 3, is created within the mixing zone 24 that aids in mixing the superabsorbent particles 11 within the fluff 10. In addition, the continual recycling of the fluff/superabsorbent particle mixture scarfed from the mold 19 back into the mixing zone 24 ensures that virtually complete mixing of the fluff 10 and superabsorbent particle 11 is achieved within the mixing zone. Consequently, the superabsorbent particles 11 are thoroughly dispersed throughout the upper layer 29 of the absorbent product 17, as shown in Figure 7.

The absorbent product 17 made by the apparatus and process described above, as shown in Figure 7, enjoys several advantages over products made by traditional approaches in which the superabsorbent particles are confined to a localized layer. Since the superabsorbent particles 11 are well dispersed throughout the product, the gel block to which localized superabsorbent particle layers are subject is much less likely to occur.

Moreover, by filling a portion of the mold cavity 20 with a layer 28 of pure fluff 10, according to the preferred embodiment of the invention, and then orienting the product so that the pure fluff layer 28 forms the body facing side 31 of the napkin, and the mixed fluff/superabsorbent particle layer 29 forms the garment facing side 32, has an advantage over products in which the superabsorbent particles 11 are dispersed throughout the entire product. Specifically, the pure fluff layer 28 is better equipped than a fluff/superabsorbent particle layer to rapidly accept large quantities of fluid directly from the body and distribute it throughout the product. After accepting the fluid, the pure fluff layer 28 can then distribute it to the fluff/superabsorbent particle layer 29 where the superior absorbing capacity, but slower absorbing rate, of the superabsorbent particles 11 can be effectively utilized.

In the event the invention were practiced utilizing heat activated fibers as the second material 11, rather than superabsorbent particles, the product of the current invention also has advantages over products made using the traditional approach. Although heat activated fibers 11 can perform a valuable function in serving to bind the fluff 10 into a unitized product, they can render the product uncomfortably stiff when confined to a localized layer, as occurs when traditional forming techniques are utilized. However, when the heat activated fibers 11 are thoroughly dispersed throughout at least a portion of the product, they not only bind the fluff fibers 10 more effectively, they do not create as large an increase in stiffness.

Filling a portion of the mold cavity 20 with a layer 28 of pure fluff 10, according to the preferred embodiment of the invention, and then orienting the product so that the pure fluff layer 28 forms the body facing side 31 of the napkin, and the mixed fluff/heat activated fiber layer 29 forms the garment facing side 32, has an advantage over products in which the heat activated fibers 11 are dispersed throughout the entire product. Specifically, the pure fluff layer 28 has the advantage of a soft feel against the user's body.

## Claims

1. An apparatus (1) for making absorbent products (17) containing first and second layers (28, 29) in which the first layer (28) contains pure first material (10) and the second layer (29) contains a mixture of the first (10) and a second (11) material, said apparatus comprising:
- a vacuum forming unit (4) including a vacuum forming chamber (12) and a forming wheel (7) within a housing (18);
- the vacuum forming chamber housing (18) having an inlet (38) being connected to a source of the first material (10) to be directed into the vacuum forming chamber (12);
- the vacuum forming chamber (12) also having an inlet (39) being connected to a source (8) of the second material (11) to be interspersed into the first material (10) within a mixing zone (24) of said vacuum forming chamber (12);
- the forming wheel (7) being mounted for positively driven rotation within the housing (18) of the vacuum forming chamber (12) and placing the vacuum forming chamber (12) in flow communication with a duct (6) through which air can be drawn by a vacuum source (5);
- a number of molds (19) being arranged circumferentially around the periphery of the forming wheel (7);
- each of the molds (19) having a cavity (20) that has the shape of the absorbent product; and
- a screen (37) being disposed in the bottom of the mold cavity (20) being connected to said vacuum source (5), if said mold (19) enters the vacuum forming chamber (12);
**characterized in that**
- said inlet (38) for the first material (10) and the inlet (39) for the second material (12) are spaced around the periphery of the vacuum forming chamber housing (18), said mixing zone (24) being formed between said first and second inlet (38; 39);
- said inlet (38) for the first material (10) is positioned at the entrance side of the vacuum forming chamber (12) for molds (19) of the forming wheel (7), so that the mold cavities (20) can be partially filled with a bottom layer (28) of pure first material (10);
- first removing means (14) being positioned, seen in the direction of rotation of the forming wheel (7), behind the entrance side of the vacuum forming chamber (12) for removing excess pure first material (10) projecting above the screen (37) of the molds (19) and directing it into the first pure material (10) so that a mold (19) being clean shaven but still only partially filled with said layer (28) of pure first material (10);
- said mixing zone (24) is spaced behind the first inlet (38) so as to completely fill the partially filled mold cavities (20) with a layer (29) of the mixture;
- second removing means (13) is spaced behind the mixing zone (24) and located so that it directs the mixture of said first and second materials (10, 11) back into the mixing zone (24) as to thoroughly disperse the second material (11) throughout the upper layer (29) of the absorbent product (17).

2. Apparatus according to claim 1, wherein said first and second removing and directing means comprise a scarfing brush (13; 14) being drivable in the same direction as the forming wheel (7).

3. Apparatus according to claim 2, wherein the scarfing brushes (13, 14) being each about 101.6 mm (four inches) in diameter and are spaced about 20° apart (about 203.2 mm (eight inches).

4. Apparatus according to any of claims 1 to 3 wherein fluff (10) made of de-fiberized wood pulp is used as the first pure material.

5. Apparatus according to any of claims 1 to 4, wherein superabsorbent particles (11) being used as the second material (11).

6. Apparatus according to any of claims 1 to 4, wherein heat activating fibers are used as the second material (11).

7. Apparatus according to claims 1 to 4, wherein baking soda is used as the second material (11).

8. Apparatus according to any of claims 1 to 5, wherein said inlet (39) for the second material (11) is pressurized.

9. Apparatus according to claim 1, wherein said forming wheel (7) has a backing plate (22) in which passages (21) are formed that place a duct (6) from the vacuum source (5) in flow communication with the molds (19) and a shaft (23) is mounted on the backing plate (22) being coupled to a motor (34) via a speed reducing device.

10. Apparatus according to claim 1, wherein said screen (37) disposed in the bottom of the mold cavity ()20) rests atop outlets of a number of air passages (35) formed within the body of the mold (19).

11. Apparatus according to claim 1, wherein a take-off wheel (15) is positioned behind the exit side of the vacuum forming chamber (12) which take-off wheel (15) is provided for removing the absorbent products (17) from the molds (19), after they have exited from the vacuum forming chamber (12) and for depositing them onto a conveyor (16) for further processing.

## Patentansprüche

1. Vorrichtung (1) zum Herstellen absorbierender Erzeugnisse (17) mit einer ersten und einer zweiten Schicht (28, 29), wobei die erste Schicht (28) ein reines erstes Material (10) und die zweite Schicht (29) ein Gemisch aus dem ersten Material (10) und einem zweiten Material (11) enthalten und die Vorrichtung umfaßt:
- eine Vakuum-Formungseinheit (4) mit einer Vakuum-Formungskammer (12) und einem Formungsrad (7) in einem Gehäuse (18);
- wobei das Vakuum-Formungskammer-Gehäuse (18) einen Einlaß (38) hat, der an eine Quelle des ersten Materials (10), das in die Vakuum-Formungskammer (12) geleitet werden soll, angeschlossen ist;
- wobei die Vakuum-Formungskammer (12) ebenfalls einen Einlaß (39) hat, der an eine Quelle (8) des zweiten Materials (11), das in einer Mischzone (24) der Vakuum-Formungskammer (12) in dem ersten Material (10) dispergiert werden soll, angeschlossen ist;
- wobei das Formungsrad (7) zu einer zwangsläufig angetriebenen Drehung in dem Gehäuse (18) der Vakuum-Formungskammer (12) montiert und dieselbe strömungsmäßig mit einer Leitung (6) verbunden ist, durch welche die Luft durch eine Vakuumquelle (5) abgesaugt werden kann;
- eine Anzahl von Formen (19), welche rund um den Umfang des Formungsrades (7) angeordnet sind;
- wobei jede dieser Formen (19) einen Hohlraum (20) hat, welcher der Form des absorbierenden Erzeugnisses entspricht und
- ein Sieb (37), das am Boden des Formhohlraumes (20) angeordnet ist und mit der Vakuumquelle (5) verbunden wird, wenn die Form (19) in die Vakuum-Formungskammer (12) eintritt;
dadurch gekennzeichnet, daß
- der Einlaß (38) für das erste Material (10) und der Einlaß (39) für das zweite Material (11) im Abstand voneinander rund um den Umfang des Vakuum-Formungskammer-Gehäuses (18) verteilt sind, und die Mischzone (24) zwischen dem ersten und dem zweiten Einlaß (38, 39) ausgebildet ist;
- der Einlaß (38) für das erste Material (10) an der Seite des Eintrittes der Formen (19) des Formungsrades (7) in die Vakuum-Formungskammer (12) angeordnet ist, so daß die Formhohlräume (20) teilweise mit einer Bodenschicht (28) des reinen ersten Materials (10) gefüllt werden können;
- eine erste Abstreifer-Einrichtung (14) in Drehrichtung des Formungsrades (7) hinter der Eintrittsseite der Vakuum-Formungskammer (12) angeordnet ist, um einen Überschuß des reinen ersten Materials (10) zu entfernen, der über das Sieb (37) der Formen (19) hinausreicht und ihn in das erste reine Material (10) zurück zu leiten, so daß die Form (19) sauber abgeschabt, aber nur teilweise mit einer Schicht (28) des ersten reinen Materials (10) gefüllt ist;
- die Mischzone (24) sich räumlich hinter dem ersten Einlaß (38) befindet, so daß die teilweise gefüllten Formhohlräume (20) hier mit einer Schicht (29) des Gemisches gefüllt werden;
- eine zweite Abstreifer-Einrichtung (13) räumlich hinter der Mischzone (24) angeordnet und derart gelegen ist, daß sie das Gemisch des ersten und zweiten Materials (10, 11) zurück in die Mischzone (24) leitet, wodurch das zweite Material (11) in der oberen Schicht (29) des absorbierenden Erzeugnisses (17) dispergiert wird.

2. Vorrichtung nach Anspruch 1, bei welcher die erste und die zweite Abstreifer- und Leit-Einrichtung aus einer Rollenbürste (13, 14) bestehen, welche in der gleichen Richtung wie das Formungsrad (7) antreibbar sind.

3. Vorrichtung nach Anspruch 2, bei welcher die Rollenbüsten (13, 14) einen Durchmesser von 101,6 mm (vier Zoll) und einen Abstand von etwa 20° oder 203,2 mm (acht Zoll) voneinander haben.

4. Vorrichtung nach einem der Ansprüche 1 bis 3, bei welcher Flocken aus von Fasern befreitem Holzschliff als erstes reines Material Verwendung finden.

5. Vorrichtung nach einem der Ansprüche 1 bis 4, bei welcher superabsorbierende Teilchen (11) als zweites Material (11) Verwendung finden.

6. Vorrichtung nach einem der Ansprüche 1 bis 4, bei welcher durch Hitze aktivierte Fasern als zweites Material (11) Verwendung finden.

7. Vorrichtung nach einem der Ansprüche 1 bis 4, bei welcher calcinierte Soda als zweites Material (11) Verwendung findet.

8. Vorrichtung nach einem der Ansprüche 1 bis 7, bei welcher der Einlaß (39) für das zweite Material (11) unter Druck steht.

9. Vorrichtung nach Anspruch 1, bei welcher das Formungsrad (7) eine Trägerplatte (22) hat, in welche Durchlässe (21) eingebracht sind, so daß eine Leitung (6) von der Vakuumquelle (5) strömungsmäßig Verbindung zu den Formen (19) hat und bei welcher eine Welle (23) an der Trägerplatte (22) angebracht ist, die über ein Reduziergetriebe an einen Motor (34) gekoppelt ist.

10. Vorrichtung nach Anspruch 1, bei welcher das am Boden des Formhohlraumes (20) angeordnete Sieb (37) auf den Auslässen einer Anzahl von Luft-Durchlässen (35) aufliegt, die im Boden der Form (19) ausgebildet sind.

11. Vorrichtung nach Anspruch 1, bei welcher ein Abnahmerad (15) hinter der Auslaßseite der Vakuum-Formungskammer (12) angeordnet ist, welches zum Entfernen der absorbierenden Erzeugnisse (17) aus den Formen (19) sowie zur Ablage derselben auf einem Förderer (16) zur weiteren Bearbeitung vorgesehen ist, nachdem diese die Vakuum-Formungskammer (12) verlassen haben.

## Revendications

1. Appareil (1) pour la production de produits absorbants (17) contenant des première et seconde couches (28, 29) dans lesquelles la première couche (28) contient une première matière pure (10), la seconde couche (29) contenant un mélange composé de la première matière (10) et d'une seconde matière (11), ledit appareil comprenant :
- un ensemble (4) de formage sous vide comprenant une chambre (12) de formage sous vide et une roue de formage (7) à l'intérieur d'un boîtier (18) ;
- le boîtier (18) de la chambre de formage sous vide ayant une entrée (38) reliée à une source de la première matière (10) à diriger dans la chambre (12) de formage sous vide ;
- la chambre (12) de formage sous vide ayant également une entrée (39) reliée à une source (8) de la seconde matière (11) devant être dispersée dans la première matière (10) à l'intérieur d'une zone de mélange (24) de ladite chambre (12) de formage sous vide
- la roue de formage (7) étant montée pour être entraînée positivement en rotation à l'intérieur du boîtier (18) de la chambre (12) de formage sous vide et pour placer la chambre (12) de formage sous vide en communication de flux avec un conduit (6) à travers lequel de l'air peut être aspiré par une source à dépression (5) ;
- un certain nombre de moules (19) agencés de façon circonférentielle autour de la périphérie de la roue de formage (7) ;
- chacun des moules (19) ayant une cavité (20) qui a la forme du produit absorbant ; et
- un tamis (37) disposé au fond de la cavité (20) du moule relié à ladite source (5) à dépression, si ledit moule (19) pénètre dans la chambre (12) de formage sous vide ;
caractérisé en ce que
- ladite entrée (38) pour la première matière (10) et l'entrée (39) pour la seconde matière (11) sont espacées autour de la périphérie du boîtier (18) de la chambre de formage sous vide, ladite zone de mélange (24) étant formée entre lesdites première et seconde entrées (38 ; 39) ;
- ladite entrée (38) pour la première matière (10) est positionnée sur le côté entrée de la chambre (12) de formage sous vide pour des moules (19) de la roue de formage (7), de sorte que les cavités (20) des moules peuvent être partiellement remplies par une couche inférieure (28) de la première matière pure (10) ;
- des premiers moyens d'enlèvement (14) sont positionnés - vus dans la direction de rotation de la roue de formage (7) - derrière le côté entrée de la chambre (12) de formage sous vide, pour enlever la première matière pure (10) en excédent formant des projections au-dessus du tamis (37) des moules (19), ladite matière en excédent étant dirigée dans la première matière pure (10), de sorte qu'un moule (19) est bien raclé mais encore seulement partiellement rempli par ladite couche (28) de la première matière pure (10) ;
- ladite zone de mélange (24) est placée à distance derrière la première entrée (38), de façon à remplir complètement les cavités (20) du moule partiellement remplies avec une couche (29) du mélange ;
- des seconds moyens d'enlèvement (13) sont à distance derrière la zone de mélange (24) et placés de façon telle qu'ils refont passer le mélange desdites première et seconde matières (10, 11) dans la zone de mélange (24), de façon à disperser complètement la seconde matière (11) dans toute la couche supérieure (29) du produit absorbant (17).

2. Appareil selon la revendication 1, dans lequel lesdits premier et second moyens d'enlèvement et directeurs comprennent une brosse de nettoyage (13 ; 14) pouvant être entraînée dans la même direction que la roue de formage (7).

3. Appareil selon la revendication 2, dans lequel les brosses de nettoyage (13, 14) ont chacune un diamètre d'environ 101,6 mm (quatre pouces) et sont espacées d'environ 20° (environ 203,2 mm (huit pouces)).

4. Appareil-selon l'une quelconque des revendications 1 à 3, dans lequel du duvet de papier (10), constitué de cellulose technique défibrée, est utilisé comme première matière pure.

5. Appareil selon l'une quelconque des revendications 1 à 4, dans lequel des particules superabsorbantes (11) sont utilisées comme seconde matière (11).

6. Appareil selon l'une quelconque des revendications 1 à 4, dans lequel des fibres thermo-actives sont utilisées comme seconde matière (11).

7. Appareil selon l'une quelconque des revendications 1 à 4, dans lequel du bicarbonate de sodium est utilisé comme seconde matière (11).

8. Appareil selon l'une quelconque des revendications 1 à 5, dans lequel ladite entrée (39) utilisée pour la seconde matière (11) est pressurisée.

9. Appareil selon la revendication 1, dans lequel ladite roue de formage (7) comprend une plaque de fixation (22) dans laquelle des passages (21) sont formés qui placent un conduit (6), depuis la source à dépression (5), en communication de flux avec les moules (19), un arbre (23) étant monté sur la plaque de fixation (22) en étant couplé à un moteur (34) via un dispositif réducteur de vitesse.

10. Appareil selon la revendication 1, dans lequel ledit tamis (37) disposé au fond de la cavité (20) du moule est placé au-dessus des sorties d'un certain nombre de passages d'air (35) formés à l'intérieur du corps du moule (19).

11. Appareil selon la revendication 1, dans lequel une roue d'enlèvement (15) est positionnée derrière le côté sortie de la chambre (12) de formage sous vide, laquelle roue d'enlèvement (15) est prévue pour que les produits absorbants (17) soient enlevés des moules (19) une fois que lesdits produits absorbants sont sortis de la chambre (12) de formage sous vide, et pour les déposer sur une bande transporteuse (16) pour la suite du processus.
